# Europäisches Patentamt

## European Patent Office

## Office européen des brevets

(19)

(11) Publication number: **0 086 649**
**B1**

(12) **EUROPEAN PATENT SPECIFICATION**

(45) Date of publication of patent specification: **30.08.89**

(21) Application number: **83300714.9**

(22) Date of filing: **11.02.83**

(51) Int. Cl.⁴: **C 12 N 15/00, C 12 P 21/02, C 07 H 21/04, C 07 K 1/00, C 12 N 1/20 // C12R1/19**

(54) Molecular cloning and characterization of the gene sequence coding for porcine relaxin.

(30) Priority: **12.02.82 AU 2695/82**

(43) Date of publication of application:
**24.08.83 Bulletin 83/34**

(45) Publication of the grant of the patent:
**30.08.89 Bulletin 89/35**

(84) Designated Contracting States:
**AT BE CH DE FR GB IT LI LU NL SE**

(56) References cited:
**EP-A-0 068 375**

**DNA, vol. 1, no. 2, 1982, Mary Ann Liebert, Inc., New York (US); J.HALEY et al.: "Porcine relaxin: Molecular cloning and cDNA structure", pp. 155-162**

**NATURE, vol. 291, May 14, 1981, Macmillan Journals Ltd, London (GB); P.HUDSON et al.: "Molecular cloning and characterization of cDNA sequences coding for rat relaxin", pp. 127-131**

(73) Proprietor: **HOWARD FLOREY INSTITUTE OF EXPERIMENTAL PHYSIOLOGY AND MEDICINE**
**c/o University of Melbourne**
**Parkville Victoria (AU)**

(72) Inventor: **Hudson, Peter John**
**1 Sefton Street**
**Bulleen Victoria (AU)**
Inventor: **Haley, John Douglas**
**Apartment 3, 29 Muir Street**
**Hawthorn Victoria (AU)**
Inventor: **Niall, Hugh David**
**3 Bendigo Avenue**
**Elwood Victoria (AU)**
Inventor: **Shine, John**
**107 Barnett Close Swinger Hill**
**Australian Capital Territory (AU)**

(74) Representative: **Brown, John David et al**
**FORRESTER & BOEHMERT Widenmayerstrasse 4/I**
**D-8000 München 22 (DE)**

(56) References cited:

CHEMICAL ABSTRACTS, vol. 96, no. 23, June 7, 1982, Columbus, Ohio (US); J.SHINE et al.: "Approaches to the cloning of relaxin gene sequences", p. 187, no. 194404k

CHEMICAL ABSTRACTS, vol. 94, no. 21, May 25, 1981, Columbus, Ohio (US); G.TREGEAR et al.: "Approaches to the solid phase synthesis of porcine relaxin", pp. 771-772, no. 175494g

CHEMICAL ABSTRACTS, vol. 97, no. 9, August 30, 1982, Columbus, Ohio (US); G.W.TREGEAR et al.: "Porcine relaxin: Synthesis and structure activity relationships", p. 675, no. 72749b

CHEMICAL ABSTRACTS, vol. 97, no. 9, August 30, 1982, Columbus, Ohio (US); G.W.TREGEAR et al.: "The synthesis of peptides with relaxin activity", p. 675, no. 72751w

**Description**

This invention relates to the molecular cloning and characterization of gene sequence codings for porcine relaxin and sub-units and equivalents thereof. The invention is also concerned with recombinant DNA techniques for the preparation of porcine relaxin, prorelaxin and preprorelaxin.

[Note literature references cited herein are given in fall at the end of the specification.]

Relaxin is a peptide hormone produced by the corpora lutea of ovaries during pregnancy. It is of interest in mammalian reproductive physiology for its critical role in the softening of the cervix and pubic symphysis prior to parturition (Hisaw, 1926). Amino acid sequence analysis has demonstrated the presence of relaxin in the ovaries of the rat (John et al., 1981), pig (Schwabe et al.; 1977; James et al., 1977) and curiously, in shark (Schwabe et al., 1978).

Like insulin, relaxin has a primary structure of two peptide chains linked by disulfide bonds, and is derived from a single chain precursor (preprorelaxin) by several processing steps. Rat preprorelaxin has been shown to contain a 105 amino acid connecting peptide linking the A and B chains (Hudson et al., 1981). Cell translations of mRNA isolated from the ovaries of pregnant pigs and immunoprecipitation of the protein products with relaxin antisera also suggested a large relaxin precursor of about 23,000 daltons as compared to 11,000 daltons for preproinsulin (Gast et al., 1980). It thus appeared likely that the pig relaxin precursor would be similar in structure to that of the rat with a large connecting peptide joining the A and B chains.

To study the structure of pig preprorelaxin we have used recombinant DNA techniques to construct *E. coli* cDNA clones containing the total mRNA population of pig ovarian corpora lutea. However, limited amino acid sequence humology exists between rat and pig relaxins (John et al., 1981) and we have found that no significant cross-hybridization occurs between the rat relaxin cDNA, available from earlier studies (Hudson et al., 1981) and the pig cDNA clones.

Consequently, we have used chemically-synthesized oligonucleotide primers to prepare pig relaxin-specific cDNA which in turn was used as a probe to isolate cDNA clones containing pig preprorelaxin coding sequences. The primary structure of the entire pig preprorelaxin coding sequence was determined from analysis of the recombinant cDNA clones.

The following abbreviations are used in this description.

DNA—deoxyribonucleic acid
RNA—ribonucleic acid
cDNA—complementary DNA (enzymatically synthesized from an mRNA sequence)
mRNA—messenger RNA
A—Adenine
T—Thymine
G—Guanine
C—Cytosine
U—Uracil

The coding relationships between nucleotide sequence in DNA and amino acid sequence is protein are collectively known as the genetic code, which is set out below.

| First position 15' end) | Second position | | | | Third position (3' end) |
|---|---|---|---|---|---|
| | U | C | A | G | |
| | Phe | Ser | Tyr | Cys | U |
| | Phe | Ser | Tyr | Cys | C |
| U | Leu | Ser | Stop | Stop | A |
| | Leu | Ser | Stop | Trp | G |
| | Leu | Pro | His | Arg | U |
| | Leu | Pro | His | Arg | C |
| C | Leu | Pro | Gln | Arg | A |
| | Leu | Pro | Gln | Arg | G |
| | Ile | Thr | Asn | Ser | U |
| | Ile | Thr | Asn | Ser | C |
| A | Ile | Thr | Lys | Arg | A |
| | Met | Thr | Lys | Arg | G |
| | Val | Ala | Asp | Gly | U |
| | Val | Ala | Asp | Gly | C |
| G | Val | Ala | Glu | Gly | A |
| | Val | Ala | Glu | Gly | G |

The abbreviations used for the amino acids in the table are identified as follows.

| | |
|---|---|
| Phenylalanine (Phe) | Lysine (Lys) |
| Leucine (Leu) | Aspartic acid (Asp) |
| Isoleucine (Ile) | Glutamic acid (Glu) |
| Methionine (Met) | Cysteine (Cys) |
| Valine (Val) | Threonine (Thr) |
| Serine (Ser) | Alanine (Ala) |
| Proline (Pro) | Tyrosine (Tyr) |
| Histidine (His) | Tryptophan (Try) |
| Glutamine (Gln) | Arginine (Arg) |
| Asparagine (Asn) | Glycine (Gly) |

Each 3-letter codon represented in the table, e.g., AUG, CAU (otherwise known as a deoxynucleotide triplet or nucleotide triplet) corresponds to a trinucleotide of mRNA, having a 5'-end on the left and a 3'-end on the right. The letters stand for the purine or pyrimidine bases forming the nucleotide sequence. All DNA sequences referred to herein are those of the strand whose sequence corresponds to the mRNA sequences described, with thymine (T) substituted for uracil (U).

The present invention has as one of its main obejctives, the provision of recombinant cDNA clones containing the structural gene sequences coding for pig preprorelaxin and peptide non-units thereof. Such clones will enable the production of porcine preprorelaxin and, hence, prorelaxin and relaxin itself, and peptide sub-units thereof, by recombinant DNA techniques.

Apart from the potential usefulness of porcine relaxin in veterinary and, possibly, human medicine, the full characterization and availability of the cDNA structure for porcine relaxin will facilitate future studies on human relaxin.

Present sources of human relaxin are limited to extracts from human tissue source material, e.g., ovaries of pregnant women and foetal membranes. The structure of human relaxin is not known and is known to differ substantially from animal relaxins. Determination of, and molecular cloning of the cDNA

4

sequence for human relaxin is thus an important research target. Gene probes based on some or all of porcine relaxin cDNA sequence may well be of value in such a determination.

In pursuance of the above objectives, we have analysed the nucleotide sequence of cloned cDNA's containing the structural gene sequence coding for pig preprorelaxin. The results allow us to predict the amino acid sequence of the signal peptide and of the connecting (C) peptide and to confirm the previously reported sequence of the B and A chains of pig relaxin. (Schwabe et al., 1977; James et al, 1977; Niall et al., 1980).

In the following description reference will be made to the accompanying drawings, in which:

Figure 1 shows a segment of the amino acid sequence of the B-chain of porcine relaxin, with the corresponding m-RNA sequence and the synthetic primer described hereinafter;

Figure 2 shows autoradiographs of gel chromatograms of cDNA sequences;

Figure 3 is the restriction endonuclease map of porcine relaxin cDNA;

Figure 4 shows the mRNA and peptide sequences of rat and pig preprorelaxin;

Figure 5 shows the mRNA and amino acid sequence for porcine preprorelaxin; and

Figure 6 shows double stranded DNA fragments used as nucleotide primers.

According to one aspect of the present invention, there is provided.

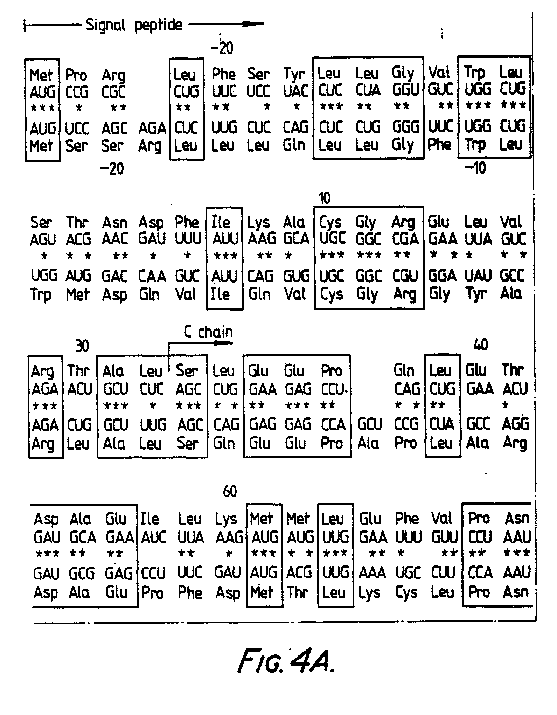

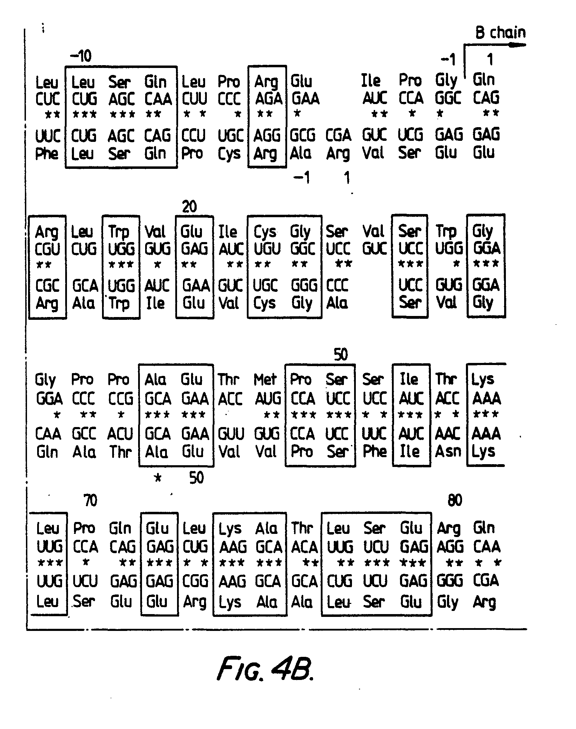

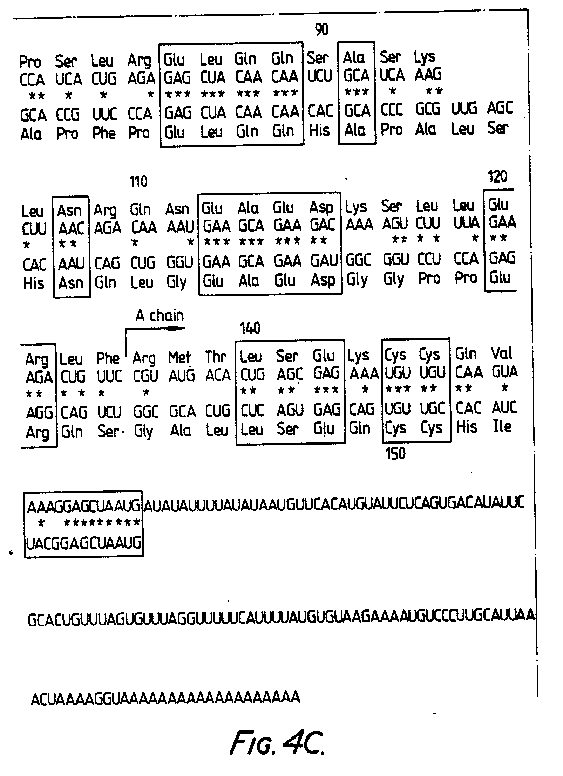

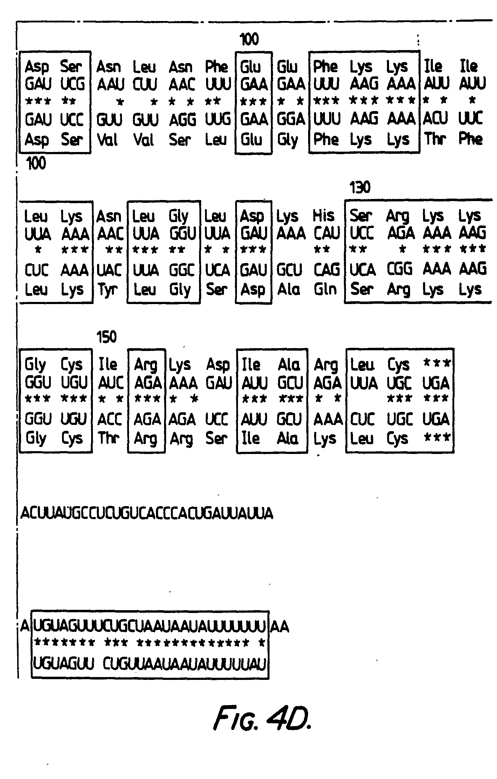

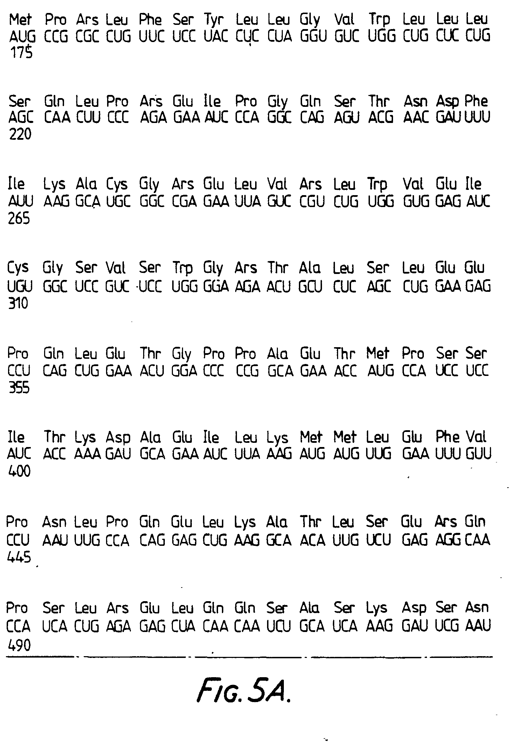

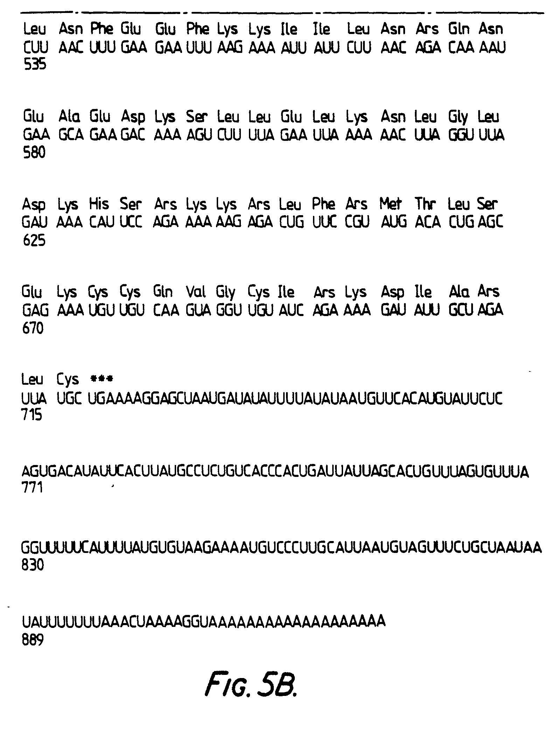

a double-stranded DNA fragment for the expression of porcine preprorelaxin, which comprises a coding strand and a complementary strand corresponding to the mRNA sequence shown in Figure 5 of the accompanying drawings.

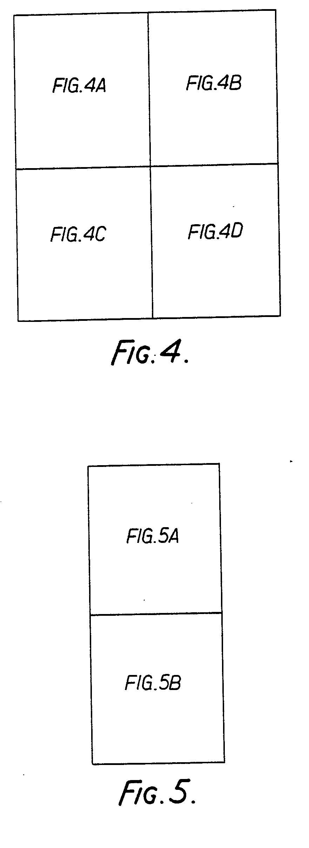

The invention also includes any sub-unit of the said sequence described herein, or any equivalent of the said sequence or sub-unit. Among the sub-units to be included by this statement are genes which exclude non-coding regions, such as that shown in Figure 5, genes containing the individual structural gene coding for the signal peptide chain and the C chain of porcine preprorelaxin (see Figure 4) and any combinations of these chains or sub-units thereof except the A plus B chain combination.

The invention further includes the complements of the above sequences, sub-units or equivalents, and the corresponding RNA sequences, sub-units or equivalents.

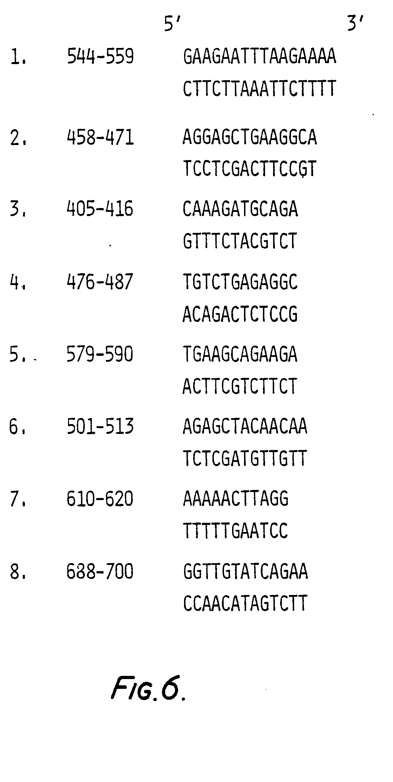

The invention also includes synthetic primers suitable for screening human or other primate relaxin genes. These double-stranded DNA fragments which are set out in Figure 6 of the accompanying drawings, correspond to the most homologous regions between the pig and rat cDNA sequences (c.f. Figure 4). The bottom strands of the sequences have been synthesized for use as nucleotide primers to screen human or other primate relaxins. The number ranges in Figure 6 indicate the corresponding sequence regions in Figure 5.

Further aspects of the invention are described hereinafter. The basis of the invention is set out in the following description of our experimental results.

Polyadenylated RNA was isolated from corpora lutea dissected from the ovaries of pigs in late pregnancy. Since cell-free translation studies of this material and readily demonstrated a product immunoprecipable by anti-relaxin antisera it was expected that relaxin-specific mRNA would be present in abundant quantities. Hence in initial studies, cDNA from "pregnant" and "non-pregnant" pig ovaries was digested with restriction endonucleases in an attempt to find predominant pregnancy-specific fragments which might derive from relaxin cDNA. As this approach was unsuccessful, it appeared that the level of relaxin cRNA was likely to be less than 1% of the total ovarian mRNA. This assumption was subsequently confirmed when it was found that the proportion of colonies containing relaxin sequences in a clone bank obtained from total pregnant corpora lutea was of the order of 0.3%.

Another approach used to identify pig relaxin cDNA clones was to screen bacterial colonies containing recombinant plasmids with a probe made from cloned rat preprorelaxin cDNA. However, specific cross-hybridization was not observed even under conditions of relatively low stringency of hybridization. This result can now be interpreted in terms of the nucleotide sequence homology between rat and pig preprorelaxin cDNA's. While the overall homology (65%) might have allowed cross-hybridization it is now known that the longest region of nucleotide sequence identity uninterrupted by mismatches is only 13 bases.

Figure 4 of the accompanying drawings shows a comparison of the porcine preprorelaxin amino acid and mRNA sequences (top) with the corresponding rat relaxin sequences (bottom). Asterisks indicate nucleotide homology, while the boxed-in areas mark amino acid homologies. Amino acids are numbered from the start of the B chain. The asterisk beneath Ala[49] denotes the putative intron processing site in rat relaxin (Hudson et al., 1981). Little nucleotide homology occurs in the 3'-untranslated region between porcine and rat relaxin mRNA's; consequently the rat nucleotide sequence is omitted from all but the boxed areas after the termination codon and surrounding the AAUAAUA sequence.

In a third approach a series of oligodeoxynucleotides were synthesized on the basis of the known amino acid sequence of pig relaxin (Scanlon, 1981).

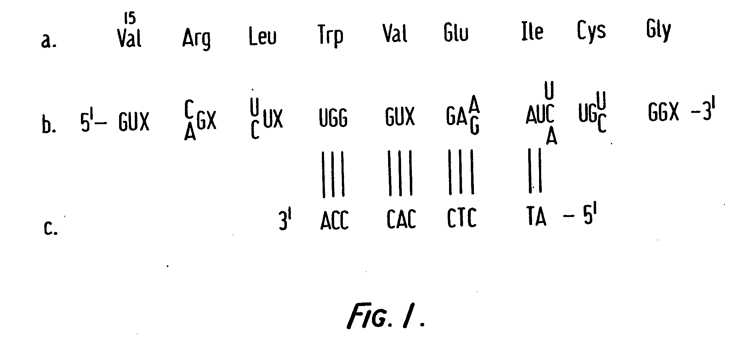

Figure 1 of the drawings shows
(a) amino acids 15 to 23 of the porcine relaxin B chain sequence which formed the basis for the synthesis of eight oligonucleotide primers which were used to hybridize specifically to porcine relaxin cRNA;

(b) the possible nucleotide sequences of the mRNA corresponding to the amino acid sequence, where X represents any base;

(c) the primer actually used to initiate relaxin specific cDNA synthesis.

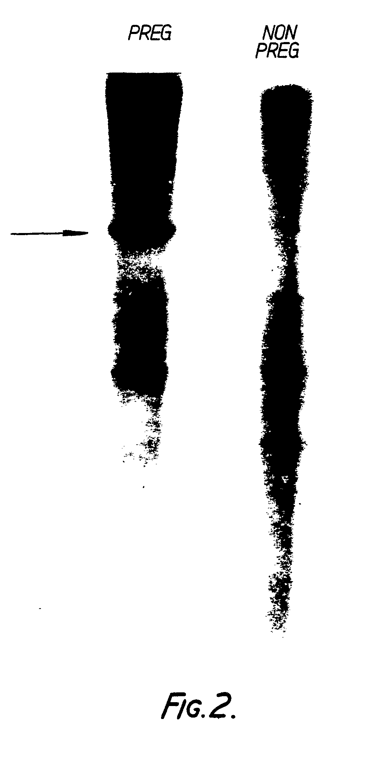

The eight 11-base primers represented by (b) for the Trp-Val-Glu-Ile region were all synthesized. Several of the primers initiated pregnancy specific cDNA products, but these proved not to contain sequences coding for porcine relaxin. However, one of the synthetic oligonucleotides [5'ATCTCCACCCA3') was found to initiate a pregnancy specific cDNA of roughly 300 nucleotides whose sequence encoded the NH$_2$-terminal region of the relaxin B chain. Using this primer cDNA was synthesized with pregnant and non-pregnant ovarian mRNA and analyzed on 6% polyacrylamide 7H urea gels. Figure 2 of the drawings shows autoradiographs of the gel columns. The arrow indicates the (approx.) 300 nucleotide $^{32}$P-labelled cDNA which was found to contain the coding sequence for the procine relaxin B chain and signal peptide. This relaxin specific cDNA was used to probe a cDNA clone bank obtained by recombinant DNA techniques from the total mRNA isolated (as described above) from pig ovaries. Approximately 5000 E. coli ovarian cDNA clones were screened by in situ filter hybridization with the relaxin-specific cDNA probe and 15 colonies proved positive on rescreening (0.3% of the total clones). Their plasmid inserts were isolated and sized on 1% agarose gels.

Several different Pst insert sizes were found, presumably caused by variation in either reverse transcription or in the SI nuclease digestion of the "hairpin" loop during the double stranding reaction. Three clones designated at pRLX2, pRLX13 and pRLX14 were selected for DNA sequence analysis.

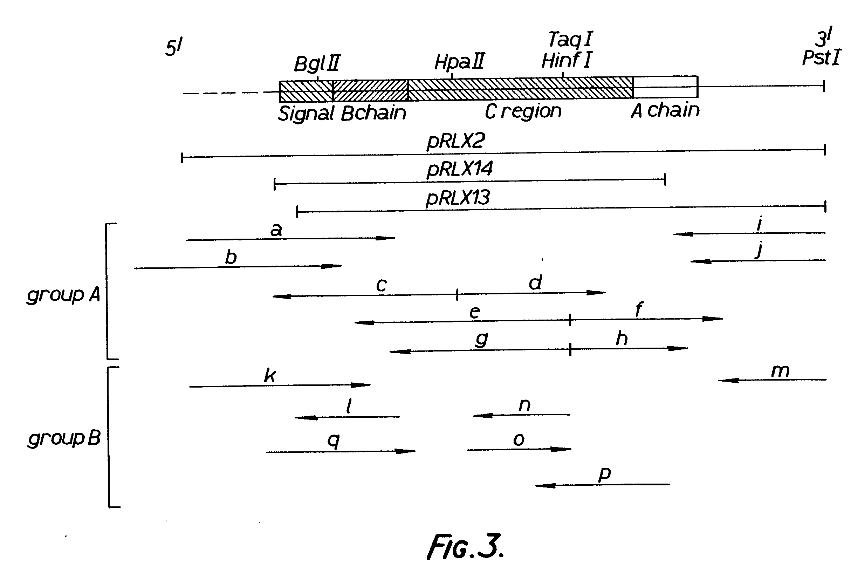

The restriction endonuclease nap of relaxin cDNA determined from analysis of these three clones appears in Figure 3 of the drawings, along with the sequencing strategy. The map also indicates the length of each clone relative to the composite structure. Group A represents those sequences derived by the Maxam and Gilbert method (the asterisk marks the position of the $^{32}$P-end label), a *PstI-HpaII; b. *HpaII-BglII; c. PstI-HpaII*; d. *HpaII-PstI; e. PstI-TaqI*; f. *TaqI-Pst; g. PstI-HinfI*; h. *HinfI-PstI; i. HinfI-PstI*; j. HinfI-HpaII*.

Group B represents those sequences derived by subcloning restriction fragments into the appropriate sites of bacteriophage M13 np 7.1 and mp 9; (k, l, m, p, q) were PstI fragments, (n, o) were HpaII-TaqI fragments subcloned into the AccI site. Recombinant plaques were sequenced using an M13-specific primer by the dideoxy procedure of Sanger et al (1980) except (l) which was primed with the relaxin-specific B-chain primer [5'ATCTCCACCCA3'].

Each of the inserts contained single HpaII and HinfI restriction sites; cleavage at these sites provided fragments for end-labelling and Maxam and Gilbert sequencing. For one of the clones, pRLX2, both DNA strands were sequenced with the exception of the A chain region and the 3' untranslated region where clear sequence was obtained using two separate restriction fragments from the same strand. The 3' untranslated region is 206 nucleotides in length beginning with the UGA termination codon at the end of the A chain. The DNA sequence of all three clones confirmed that the nucleotide sequence encoded the known A and B chain primary structure of porcine relaxin (Fig. 4). Regions corresponding to a C or connecting peptide and a signal peptide could be identified, giving an overall structure of the form: signal peptide—B chain—C peptide—A chain.

The invention further provides, a DNA transfer vector as defined above in which one or more material codons or their cDNA equivalents are replaced by another codon which codes for the same amino-acid, or in which one or more of the natural codons are deleted and/or are replaced by codons which code for amino acids other than that code by the natural codon, and/or further codons are added to the natural sequence.

The DNA transfer vector may be a bacterial plasmid bacteriophage DNA.

Thus, in one specific aspect the invention may provide a plasmid recombinant which comprises a plasmid vector having inserted therein at an insertion site a gene or a sub-unit thereof or an equivalent thereof, according to the above-described embodiments, the plasmid recombinant enabling translation in the correct phase for the mRNA corresponding to the inserted gene or sub-unit thereof or equivalent thereof and having a bacterial promoter upstream of and adjacent to the insertion site such that the inserted gene or sub-unit thereof or equivalent thereof is under bacterial promoter control.

The production of such a recombinant molecule by a process which comprises inserting DNA corresponding to the desired structural gene for relaxin at the insertion site of an appropriate vector also constitutes an embodiment of the present invention.

The present invention further provides a transformed cell which comprises inserted therein such a gene or a sub-unit thereof or an equivalent thereof or such a plasmid recombinant.

The production of such a cell by a process which comprises inserting the DNA via a suitable vector into a cell also constitutes an embodiment of the present invention.

In a further embodiment, the present invention provides a process for the production of porcine preprorelaxin, or a protein having properties resembling those of porcine preprorelaxin, which comprises culturing such a cell and recovering expressed protein.

The invention also includes the production of porcine prorelaxin or the relaxin C peptide by analogous methods to those described above, which may also involve synthetic modification of the cDNA, according to methods and practices already known in the art.

The invention also extends to synthetic preprorelaxin, prorelaxin and the C peptide chain, especially when prepared by the above-described processes.

The invention further includes the production of relaxin from preprorelaxin or prorelaxin, or by joining of the A and B chains by methods analogous to those used for the production of insulin from its respective precursors.

The materials and procedures used in the methods associated with or forming part of the invention are further illustrated by the following descriptions.

Materials

Terminal transferase was purchased from Ratcliff Biochemicals (Los Alamos, N. M.) Pst I, Bgl II, DNA polymerase (Klenow fragment) and SI nuclease were from Boehringer Mannheim, Hinf I, TaQ I, Hae III, Alu I, and $T_4$ DNA ligase were from New England Biolabs (Beverly, MA). Oligo-dT cellulose type 7 was obtained from PL Biochemicals (Milwaukee, Wis). [12]P-labelled nucleoside triphosphates and $^{35}$S-labelled amino acids were from Amersham.

Messenger RNA isolation

Ovaries from pigs in late pregnancy were obtained by collection at abattoirs and quickly frozen on dry ice. The corpora lutea were disected free of other tissue, the RNA isolated in 5M guanidinium thiocyanate (Merck) (Chirgwin et al., 1979) and the poly $A^+$ containing RNA purified by oligo—dT cellulose affinity chromatography (Aviv and Leder, 1972). Yields were approximately 10 µg poly $A^+$ RNA per 100 mg tissue. To assay mRNA for relaxin-containing message, cell free translations were carried out employing rabbit reticulocyte lysate (Pelham and Jackson, 1976) and immunoprecipitated proteins (Kessler, 1975) were electrophoresed on SDS polyacrylamide gels (Studier, 1973).

cDNA was prepared using reverse transcriptase and an oligo—dT primer (Wickers et al., 1978), the RNA hydrolyzed in alkali, and the cDNA made double-stranded (Ullrich et al., 1977) with DNA polymerase I (Klenow fragment). The single-stranded hairpin loop was removed with 10 units of SI nuclease at 20°C for 5' (Vogt, 1973).

Homopolymeric deoxycytidine extensions were emzymatically added using terminal transferase (10 units in 50 µl reaction volume at 37° for 10') in a controlled reaction designed to add approximately 30 C-residues to the 3'-OH ends (Roychoudbury et al., 1976). This "tailed" cDNA was annealed to the plasmid pBR322 which had been cleaved with PstI and similarly "tailed" with G-residues. The recombinant plasmids were used to transform *E. coli* RRI (efficiency=$10^5$ clones per µg plasmid) and clones containing ovarian cDNA were selected on the basis of tetracycline resistance (Morrison, 1979).

Selection of specific cDNA clones

To screen the ovarian cDNA clone bank for relaxin specific sequences, eight 11-base primers were chemically synthesized by the phosphotriester method (Itakura et al., 1973) to make all possible complementary sequences corresponding to the peptide sequence Trp-Val-Glu-Ile in the pig relaxin B chain (Fig. 1). These primers were used to select specific cDNA products for use as hybridization probes to the cDNA clone bank.

Oligonucleotide primers were used to initiate reverse transcription of mRNA under conditions designed for the synthesis of high specific activity and yield full length cDNA. 2 µg of mRNA was added to between 0.1 and 1 µg of primer, heated to 90°C for 2 minutes, slowly cooled, and the reverse transcriptase reaction carried out at 37°C in 50 mM Tris pH 8.0, 50 mM HaCl, 10mM $MgSO_4$, 0.1mM DTT with 600µM dATP, dTTP, dGTP, 30µM dCTP, 30 Ci β-$^{32}$P-dCTP, and 10 units of reverse transcriptase. Reaction products were alkali treated (100mM NaOH, 80°C, 10 min) to remove RNA and analyzed on 6% acrylamide 7M urea gels for the presence of pregnancy-specific cDNA's (Fig. 2). In order to identify which of the cDNA clones contained relaxin-specific sequences in a separate experiment the primer (lacking the 5' phosphate) was radiochemically end-labelled with T4 polynucleotide kinase and γ$^{32}$P-ATP (100 Ci per µg primer) and the cDNA synthesized in the absence of β-$^{32}$P-deoxynucleotide (Noyes et al. 1979). This yielded end-labelled DNA suitable for sequencing by the chemical degradation technique (Maxam and Gilbert, 1977). The regions of the gel correponding to specific cDNA bands were excised, placed in dialysis membrane bags and the DNA electrophoretically removed from the gel matrix in 50mM Tris-borate, 1mM EDTA (pH 8.1). Eluted DNA samples were precipitated with 2 volumes of ethanol in 0.3M Na acetate (pH 5.5) at −20°C for 4 hours.

The relaxin-specific cDNA fragment identified by the methods described above was synthesized to a specific activity greater than $10^8$ cpm/µg, purified on polyacrylamide gels, and used to screen the cDNA clone banks (Thayer, 1979). Colonies which hybridized to this probe were rescreened in duplicate in order to grade the positives as to their rate of specific hybridization. The strongest hybridizing clones were screened by a rapid plasmid isolation procedure (Homes and Quigley, 1981) and those containing the largest recombinant plasmids were grown in one litre cultures for large scale plasmids isolation (Tanaka and Weisblum, 1975) yielding about 700 µg of plasmid.

Sequence analysis of recombinant plasmids

Restriction digests were carried out under the conditions specified by New England Biolabs. Purified

7

DNA fragments with 5' protruding ends were end-labelled using reverse transcriptase and the appropriate β-labelled deoxynucleotide triphosphase (dCTP for Hpall and Taql; dATP for Hinfl, 3'-protruding ends generated with PstI were labelled using terminal transferase and [$^{32}$P]-cordycepin triphosphate. Fragments were separated by electrophoresis on 8% polyacrylamide gels and sequenced by chemical degradation method (Maxam and Gilbert, 1977).

The cloned cDNA inserts released by Pst I digestion were also ligated into bacteriophase M13 mp9 or mp7.1 and the single-stranded template DNA isolated and sequenced using the dideoxy procedure (Sanger et al. 1980). The M13 recombinants were sequenced using either the M13-specific primer or with specific primers chosen over particular regions of the internal relaxin sequence.

Expression of porcine preprorelaxin

The expression vector TrpED5-1 (Dallewell, R. A. and Emtage, S., 1980, *Gene 9*, 27) contains the replicative properties of pBR322 with the tryptophan promoter, and TrpE and TrpD sequences. The preprorelaxin gene may be introduced into the TrpD sequence via a unique Hind III restriction endonuclease site, producing a fusion TrpD/preprorelaxin protein regulated by the inducible Trp promoter.

TrpED5-1 was cleaved with Hind III 37°C for 3 hours, (50mM Tris Cl pH 7.4, 10mM MgCl$_2$, 50 mM NaCl), and the enzyme inactivated at 70°C for 10 minutes, and the staggered ends filled in with T4 Polymerase (200 mM dATP, dCTP, dGTP, dTTP; 11°C 3 hrs). The preprorelaxin sequence (Haley, J. D. *et al* 1982) DNA 1, 155—162) was cleaved with Xba to remove the TAG termination codon in the 5' non-translated sequences, The 3' end was cleaved with Pst and was full length. The staggered termini were removed with 10 units of SI nuclease 20°C 5 mintues (0.3M NaCl, 20mM NaAc pH 7.6, 3mM ZnAc) to produce blunt ends. The reaction was stopped by phenol/chloroform extraction, ether extraction and ethanol precipitation of the supernatant. Equimolar amounts of processed TrpED5-1 vector and preprorelaxin were joined with T4 ligase (10°C 24 hours, 50 mM Tris·Cl pH7.4, 10mM NgCl$_2$, 1mM DTT, 1mM ATP).

E. coli 294 grown to ED$_{600}$=0.3 were made competent by CaCl$_2$ treatment (75mM CaCl$_2$, 10mM Tris·Cl pH7.4, 4°C 1 hour). Joined TrpED5-1/preprorelaxin DNA was added. After 3 hours on ice the cell/DNA mix was heat shocked (45°C 90 sec) and the mix returned to the ice for an additional 30 minutes. Cells were plated on L broth plates supplemented with 20μg/ml Ampicillin, and recombinant bacteria were screened with β-$^{32}$P-dATP labelled preprorelaxin by *in situ* filter hybridization (Thayer, R. E. (1979), Anal.Biochem, 98, 60—63). Recombinant bacteria produced in this manner are capable of producing TrpD/preprorelaxin fusion protein inducible with 3-indolacrylic acid (Martial, J. A. *et al* (1979) Science 205, 602-6-6).

References

Aviv, H. and Leder, P. (1972). Purification of biologically active globin messenger RNA by chromatography on oligothymidylic acid-cellulose, Proc. Nat. Acad. Sci. USA 69, 1408—1412.

Chirgwin, J. M., Przybyla, A. E., MacDonald, R. J. and Rutter, W. J. (1979) Isolation of biologically active ribonucleic acid from sources enriched in ribonuclease. Biochem. 18, 5294—5299.

Gast, M. J., Mercado-Simmen, R., Niall, H, and Boime, I. (1980) Cell-free synthesis of a high molecular weight relaxin-related protein, Ann. N.Y. Acad. Sci. 343, 148—154.

Hackenthal, E., Hackenthal, R. and Hilgenfeldt, U. (1978) Isorenin, pseudorenin, cathepsin D and renin: a comparative enzymatic study of angiotensin-forming enzymes, Biochim. Biophys. Acta 522, 574—588.

Hisaw, F.L. (1926) Experimental relaxation of the pubic ligament of the guineapig. Proc. Soc. Exp. Bio. Med. 23, 661—663.

Holmes, D.S. and Quigley, M. (1981) A rapid boiling method for the preparation of bacterial plasmids. Anal Biochem. 114. 198—197.

Hudson, P., Haley, J., Cronk, M., Shine, J. and Niall, H. (1981) Molecular cloning and characterization of cDNA sequences coding for rat relaxin. Nature 291, 127—131.

Itakura, K., Bahl, C.P., Katagiri, N., Michniewicz, J.J., Wrightman, R.H. and Narang, S. A. (1973) A modified triester method for the synthesis of deoxypolynucleotides, Can. J. Chem. 51, 3649—3651.

James, R., Niall, H., Kwok, S. and Bryant-Greenwood, G. (1977) Primary structure of porcine relaxin: homology with insulin and related growth factors, Nature 267, 544—546.

John, M.J., Walsh, J.R., Borjesson, B.W. and Niall, H.D. (1981) Limited sequence homology between porcine and rat relaxin: implications for physiological studies. Endocrinology 108, 726—729.

Kessler, S.W. (1975) Rapid isolation of antigens from cells with a staphylococcal protein A-antibody adsorbant: parameters of the interaction of antibody-antigen complex with protein A./J. Immunol, 115, 1617—1624.

Maxam, A.N. and Gilbert, W. (1977) A new method for sequencing DNA. Proc. Natl. Acad. Sci. USA 74, 560—564.

Mercier, J—C., Brignon, G. and Ribadeau-Dumas B. (1973) Primary structure of bovine kB-casein. Eur. J. Biochem 35, 222—235.

McDonald, J.K., Callahan, P.X., Ellis, S. and Smith, R.E. (1977) In: Tissue Proteinases. A.J. Barret and J.T. Dingle, eds. (Amsterdam: North-Holland Publishing Co.) pp. 69—107.

Morrison, D.A. (1979) In: Methods in Enzymology, R. Wu, ed. (New York: Academic Press) pp. 326—331.

Niall, H.D., James, R., John, M., Walsh, J., Kwok, S., Bryant-Greenwood, G.D., Tregear, G.W. and Bradshaw, R.A. (1980) Proceedings of the 15th Midwest Conference on Endocrinology and Metabolism (New York: Plenum Publishing) (in press).

Noyes, B.E., Mevarech, M., Stein, R. and Agarwal, K.L. (1979) Detection and partial sequence analysis of gastrin mRNA by using an oligonucleotide probe, Proc. Natn. Acad. Sci. 76, 1770—1774.

Pelham, H.R.B. and Jackson, R.J. (1976) An efficient mRNA-dependent translation system from reticulocyte lysates. Eur. J. Biochem. 67, 247—256.

Proudfoot, N.J. and Brownlee, G.G. (1976) 3' non-coding region sequences in eukaryotic messenger RNA. Nature 263, 211—214.

Roychoudbury, R. Jay, E. and Wu, R. (1976) Terminal labelling and addition of homopolymer tracts to duplex DNA fragments by terminal deoxynucleotidyl transferase. Nucleic Acid Res. 3, 863—877.

Sanger, F., Coulson, A.R., Barrell, B.G., Smith, A.J.H. and Roe B.A. (1980) Cloning in single stranded bacteriophage as an aid to rapid DNA sequencing. J. Mol. Biol. 143, 161—178.

Schwabe, C., McDonald, J.K. and Steinetz, B.C. (1977) Primary structure of the B-chain of porcine relaxin Biochem biophys. Res. Commun, 75, 503—510.

Schwabe, C., Steinetz, B., Weise, G., Segaloff, A. McDonald, J.K., O'Byrne, E., Hochman, J., Carrier, B. and Goldsmith, L. (1978) Relaxin, Rec. Progr. Horm. Res. 34, 123—211.

Sherwood, O.D. and O'Byrne, E.M. (1974) Purification and characterization of porcine relaxin. Arch. Biochem. Biophys. 160, 185—196.

Steiner, D.F., Quinn, P.S., Chan, S.J., Marsh, J. and Tager, H.S. (1980) Processing mechanisms in the biosynthesis of proteins. Ann N.Y. Acad. Sci. 343, 1—16.

Studier, F.W. (1973) Analysis of bacteriophage T7 early RNAs and proteins on slab gels. J. Mol. Biol. 79, 237—248.

Tager, H. S., Emdin, S.O., Clark, J.L. and Steiner, D.F. (1973). Studies on the conversion of proinsulin to insulin, J. Biol. Chem. 248, 3476—3482.

Tanaka, T. and Weisblum, B. (1975) Construction of a colicin EI-R factor composite plasmid in vitro: means for amplification of deoxy-ribonucleic acid, J. Bacteriol, 121, 354—362.

Thayer, R.E. (1979) An improved method for detecting foreign DNA in plasmids of Escherichia coli Anal. Biochem 98, 60—63.

Thibault, G. and Genest, J. (1981) Tonin, an esteroprotease from rat submaxillary glands. Biochimica et Biophysica Acta 660, 23—29.

Ullrich, A., Shine, J., Chirgwin, J., Picket, R., Tischer, E., Rutter, W.J. and Goodman, H.M. (1977) Rat insulin genes: construction of plasmids containing the coding sequences. Science 196, 1313—1319.

Vogt, V.M. (1973) Purification and further properties of single-strand-specific nuclease from Aspergillus oryzae. Eur. J. Biochem. 33, 192—200.

Walsh, J.R. and Niall, H.D. (1980) Use of an octadecylsilica purification method minimizes proteolysis during isolation of porcine and rat relaxins. Endocrinology 107, 1258—1260.

Wickers, M.P., Buell, G.N. and Schinke, R.T. (1978) Synthesis of double-stranded DNA complementary to lysozyme, ovomucoid, and ovalbumin mRNAs. J. Biol. Chem. 253, 2483—2495.

**Claims**

1. A DNA fragment coding for porcine preprorelaxin, characterized in that it comprises a coding strand and a complementary strand corresponding to the following complete mRNA sequence:

```
AUG CCG CGC CUG UUC UCC UAC CUC CUA GGU GUC UGG CUG CUC
CUG AGC CAA CUU CCC AGA GAA AUC CCA GGC CAG AGU ACG AAC
GAU UUU AUU AAG GCA UGC GGC CGA GAA UUA GUC CGU CUG UGG
GUG GAG AUC UGU GGC UCC GUC UCC UGG GGA AGA ACU GCU CUC
AGC CUG GAA GAG CCU CAG CUG GAA ACU GGA CCC CCG GCA GAA
ACC AUG CCA UCC UCC AUC ACC AAA GAU GCA GAA AUC UUA AAG
AUG AUG UUG GAA UUU GUU CCU AAU UUG CCA CAG GAG CUG AAG
GCA ACA UUG UCU GAG AGG CAA CCA UCA CUG AGA GAG CUA CAA
CAA UCU GCA UCA AAG GAU UCG AAU CUU AAC UUU GAA GAA UUU
AAG AAA AUU AUU CUU AAC AGA CAA AAU GAA GCA GAA GAC AAA
AGU CUU UUA GAA UUA AAA AAC UUA GGU UUA GAU AAA CAU UCC
AGA AAA AAG AGA CUG UUC CGU AUG ACA CUG AGC GAG AAA UGU
UGU CAA GUA GGU UGU AUC AGA AAA GAU AUU GCU AGA UUA UGC
UGAAAAGGAGCUAAUGAUAUAUUUUAUAUAAUGUUCACAUGUAUUCUCAGUGACA
UAUUCACUUAUGCCUCUGUCACCCACUGAUUAUUAGCACUGUUUAGUGUUUAGGU
UUUUCAUUUUUUGUGUAAGAAAAUGUCCCUUGCAUUAAUGUAGUUUCUGCUAAUA
AUAUUUUUUUAAACUAAAAGGUAAAAAAAAAAAAAAAAAAAA.
```

2. A DNA fragment coding for porcine prorelaxin, characterized in that it comprises a coding strand and a complementary strand corresponding to the following mRNA sequence:

```
CAG AGU ACG AAC
GAU UUU AUU AAG GCA UGC GGC CGA GAA UUA GUC CGU CUG UGG
GUG GAG AUC UGU GGC UCC GUC UCC UGG GGA AGA ACU GCU CUC
```

AGC CUG GAA GAG CCU CAG CUG GAA ACU GGA CCC CCG GCA GAA
ACC AUG CCA UCC UCC AUC ACC AAA GAU GCA GAA AUC UUA AAG
AUG AUG UUG GAA UUU GUU CCU AAU UUG CCA CAG GAG CUG AAG
GCA ACA UUG UCU GAG AGG CAA CCA UCA CUG AGA GAG CUA CAA
CAA UCU GCA UCA AAG GAU UCG AAU CUU AAC UUU GAA GAA UUU
AAG AAA AUU AUU CUU AAC AGA CAA AAU GAA GCA GAA GAC AAA
AGU CUU UUA GAA UUA AAA AAC UUA GGU UUA GAU AAA CAU UCC
AGA AAA AAG AGA CUG UUC CGU AUG ACA CUG AGC GAG AAA UGU
UGU CAA GUA GGU UGU AUC AGA AAA GAU AUU GCU AGA UUA UGC.

3. A double-stranded DNA fragment coding for the signal peptide chain of porcine preprorelaxin, characterized in that it comprises a coding strand and a complementary strand corresponding to the following mRNA sequence:

AUG CCG CGC CUG UUC UCC UAC CUC CUA GGU GUC UGG CUG CUC
CUG AGC CAA CUU CCC AGA GAA AUC CCA GGC.

4. A double-stranded DNA fragment coding for the C peptide chain of porcine preprorelaxin, characterized in that it comprises a coding strand and complementary strand corresponding to the following mRNA sequence:

AGC CUG GAA GAG CCU CAG CUG GAA ACU GGA CCC CCG GCA GAA
ACC AUG CCA UCC UCC AUC ACC AAA GAU GCA GAA AUC UUA AAG
AUG AUG UUG GAA UUU GUU CCU AAU UUG CCA CAG GAG CUG AAG
GCA ACA UUG UCU GAG AGG CAA CCA UCA CUG AGA GAG CUA CAA
CAA UCU GCA UCA AAG GAU UCG AAU CUU AAC UUU GAA GAA UUU
AAG AAA AUU AUU CUU AAC AGA CAA AAU GAA GCA GAA GAC AAA
AGU CUU UUA GAA UUA AAA AAC UUA GGU UUA GAU AAA CAU UCC
AGA AAA AAG AGA CUG UUC.

5. A DNA fragment encoding an allelic variant of the DNA fragments claimed in any one of Claims 1, 2, 3 and 4.

6. A DNA fragment coding for the signal or C peptide chains of porcine preprorelaxin or any combination of two or more of the signal A, B or C peptide chains of porcine preprorelaxin, excluding the A plus B combination, the peptide chains being as defined in the foregoing claims.

7. A process for the production of a DNA fragment according to any one of the preceding claims, characterized in that it comprises using recombinant DNA techniques to obtain a cDNA clone bank from total mRNA isolated from porcine corpora lutea, and screening the clone bank using a cDNA probe initiated by the synthetic oligonucleotide [5'ATCTCCACCCA3'].

8. A DNA transfer vector, characterized in that it contains a cDNA deoxynucleotide sequences corresponding to a DNA fragment according to any one of Claims 1 to 6.

9. A DNA fragment or DNA transfer vector as claimed in any one of Claims 1 to 6 and 8, characterized in that one or more natural codons or their cDNA equivalents are replaced by another codon which codes for the same amino-acid.

10. A DNA transfer vector according to Claim 8 or 9, characterized in that it is a bacterial plasmid.

11. A DNA transfer vector according to Claim 8 or 9, which is bacteriophage DNA.

12. A cell transformed by a transfer vector according to Claim 8 or 9.

13. A process for making a DNA transfer vector for use in maintaining and replicating a DNA fragment coding for porcine preprorelaxin according to any one of Claims 1, 5 and 9, characterized in that it comprises reacting the appropriate deoxynucleotide sequence of porcine preprorelaxin or allelic variant with a DNA molecule prepared by cleaving a transfer vector with a restriction enzyme.

14. A process for making a DNA transfer vector according to Claim 8 or 9, for use in maintaining and replicating a DNA fragment coding for porcine prorelaxin, characterized in that it comprises reacting the appropriate DNA fragment with a DNA molecule prepared by cleaving a transfer vector with a restriction enzyme.

15. A process for making a DNA transfer vector according to Claim 8 or 9, for use in maintaining and replicating a DNA fragment coding for the signal peptide or the C chain peptide of porcine preprorelaxin or any combination of two or more of the signal, A, B or C chain peptides, except the A plus B chains combination characterized in that it comprises reacting the appropriate DNA fragment with a DNA molecule prepared by cleaving a transfer vector with a restriction enzyme.

16. Porcine preprorelaxin, having the amino acid sequence

Met Pro Arg Leu Phe Ser Tyr Leu Leu Gly Val Trp Leu Leu Leu Ser Gln Leu Pro Arg Glu Ile Pro
Gly Gln Ser Thr Asn Asp Phe Ile Lys Ala Cys Gly Arg Glu Leu Val Arg Leu Trp Val Glu Ile Cys
Gly Ser Val Ser Trp Gly Arg Thr Ala Leu Ser Leu Glu Glu Pro Gln Leu Glu Thr Gly Pro Pro Ala
Glu Thr Met Pro Ser Ser Ile Thr Lys Asp Ala Glu Ile Leu Lys Met Met Leu Glu Phe Val Pro Asn
Leu Pro Gln Glu Leu Lys Ala Thr Leu Ser Glu Arg Gln Pro Ser Leu Arg Glu Leu Gln Gln Ser Ala
Ser Lys Asp Ser Asn Leu Asn Phe Glu Glu Phe Lys Lys Ile Ile Leu Asn Arg Gln Asn Glu
Ala Glu Asp Lys Ser Leu Leu Glu Leu Lys Asn Leu Gly Leu Asp Lys His Ser Arg Lys Lys Arg Leu
Phe Arg Met Thr Leu Ser Glu Lys Cys Cys Gln Val Gly Cys Ile Arg Lys Asp Ile Ala Arg Leu Cys

17. Porcine prorelaxin, having the amino acid sequence

Gln Ser Thr Asn Asp Phe Ile Lys Ala Cys Gly Arg Glu Leu Val Arg Leu Trp Val Glu Ile Cys
Gly Ser Val Ser Trp Gly Arg Thr Ala Leu Ser Leu Glu Glu Pro Gln Leu Glu Thr Gly Pro Pro Ala
Glu Thr Met Pro Ser Ser Ile Thr Lys Asp Ala Glu Ile Leu Lys Met Met Leu Glu Phe Val Pro Asn
Leu Pro Gln Glu Leu Lys Ala Thr Leu Ser Glu Arg Gln Pro Ser Leu Arg Glu Leu Gln Gln Ser Ala
Ser Lys Asp Ser Asn Leu Asn Phe Glu Glu Phe Lys Lys Ile Ile Leu Asn Arg Gln Asn Glu
Ala Glu Asp Lys Ser Leu Leu Glu Leu Lys Asn Leu Gly Leu Asp Lys His Ser Arg Lys Lys Arg Leu
Phe Arg Met Thr Leu Ser Glu Lys Cys Cys Gln Val Gly Cys Ile Arg Lys Asp Ile Ala Arg Leu Cys

18. A process for making a fusion protein comprising an amino acid sequence consisting of the amino acid sequence of porcine preprorelaxin according to Claim 16 as its C-terminal sequence and a portion of a procaryotic protein as its N-terminal sequence, characterized in that it comprises incubating a microorganism transformed by an expression transfer vector according to claims 8 or 9 comprising the appropriate DNA fragment, according to claims 1 or 5.

19. A process for synthesizing porcine prorelaxin according to Claim 17 comprising the A and B peptides separated from each other by a C peptide, characterized in that it comprises incubating a microorganism, transformed by an expression transfer vector according to claims 8 or 9 comprising a DNA fragment coding for said porcine prorelaxin according to claims 2 or 5 under conditions suitable for expression of said sequence coding for porcine prorelaxin, and purifying porcine prorelaxin from the lysate or culture medium of said microorganism.

20. A fusion protein comprising an amino acid sequence characterized in that it consists of all or part of the amino acid sequence of porcine preprorelaxin according to Claim 16 as its C-terminal sequence and a portion of a procaryotic protein as its N-terminal sequence, with the proviso that the amino acid sequence is not solely the A-chain or the B chain sequence or porcine relaxin or the A plus B chain combination.

21. The synthetic signal peptide chain of porcine prorelaxin having the sequence

Met Pro Arg Leu Phe Ser Tyr Leu Leu Gly Val Trp Leu
Leu Leu Ser Gln Leu Pro Arg Glu Ile Pro Gly.

22. The synthetic C peptide chain of porcine relaxin having the sequence

Ser Leu Glu Glu Pro Gln Leu Glu Thr Gly Pro Pro Ala
Glu Thr Met Pro Ser Ser Ile Thr Lys Asp Ala Glu Ile Leu Lys Met Met Leu Glu Phe Val Pro Asn
Leu Pro Gln Glu Leu Lys Ala Thr Leu Ser Glu Arg Gln Pro Ser Leu Arg Glu Leu Gln Gln Ser Ala
Ser Lys Asp Ser Asn Leu Asn Phe Glu Glu Phe Lys Lys Ile Ile Leu Asn Arg Gln Asn Glu
Ala Glu Asp Lys Ser Leu Leu Glu Leu Lys Asn Leu Gly Leu Asp Lys His Ser Arg Lys Lys Arg Leu
Phe

## Patentansprüche

1. DNA-Fragment, das für Schweine-Präprorelaxin codiert, dadurch gekennzeichnet, daß es einen codierenden Strang und einen komplementären Strang aufweist, entsprechend der nachfolgenden vollständigen mRNA-Sequenz:

AUG CCG CGC CUG UUC UCC UAC CUC CUA GGU GUC UGG CUG CUC
CUG AGC CAA CUU CCC AGA GAA AUC CCA GGC CAG AGU ACG AAC
GAU UUU AUU AAG GCA UGC GGC CGA GAA UUA GUC CGU CUG UGG
GUG GAG AUC UGU GGC UCC GUC UCC UGG GGA AGA ACU GCU CUC
AGC CUG GAA GAG CCU CAG CUG GAA ACU GGA CCC CCG GCA GAA
ACC AUG CCA UCC UCC AUC ACC AAA GAU GCA GAA AUC UUA AAG
AUG AUG UUG GAA UUU GUU CCU AAU UUG CCA CAG GAG CUG AAG
GCA ACA UUG UCU GAG AGG CAA CCA UCA CUG AGA GAG CUA CAA
CAA UCA GCA UCA AAG GAU UCG AAU CUU AAC UUU GAA GAA UUU
AAG AAA AUU AUU CUU AAC AGA CAA AAU GAA GCA GAA GAC AAA
AGU CUU UUA GAA UUA AAA AAC UUA GGU UUA GAU AAA CAU UCC
AGA AAA AAG AGA CUG UUC CGU AUG ACA CUG AGC GAG AAA UGU
UGU CAA GUA GGU UGU AUC AGA AAA GAU AUU GCU AGA UUA UGC
UGAAAAGGAGCUAAUGAUAUAUUUUAUAUAAUGUUCACAUGUAUUCUCAGUGACA
UAUUCACUUAUGCCUCUGUCACCCACUGAUUAUUAGCACUGUUUAGUGUUUAGGU
UUUUCAUUUUAUGUGUAAGAAAAUGUCCCUUGCAUUAAUGUAGUUUCUGCUAAUA
AUAUUUUUUUAAACUAAAAGGUAAAAAAAAAAAAAAAAAAAA

2. DNA-Fragment, das für Schweine-Prorelaxin codiert, dadurch gekennzeichnet, daß es einen codierenden Strang und einen komplementären Strang aufweist, entsprechend der folgenden mRNA-Sequenz:

CAG AGU ACG AAC
GAU UUU AUU AAG GCA UGC GGC CGA GAA UUA GUC CGU CUG UGG
GUG GAG AUC UGU GGC UCC GUC UCC UGG GGA AGA ACU GCU CUC
AGC CUG GAA GAG CCU CAG CUG GAA ACU GGA CCC CCG GCA GAA
ACC AUG CCA UCC UCC AUC ACC AAA GAU GCA GAA AUC UUA AAG
AUG AUG UUG GAA UUU GUU CCU AAU UUG CCA CAG GAG CUG AAG

EP 0 086 649 B1

GCA ACA UUG UCU GAG AGG CAA CCA UCA CUG AGA GAG CUA CAA
CAA UCA GCA UCA AAG GAU UCG AAU CUU AAC UUU GAA GAA UUU
AAG AAA AUU AUU CUU AAC AGA CAA AAU GAA GCA GAA GAC AAA
AGU CUU UUA GAA UUA AAA AAC UUA GCU UUA GAU AAA CAU UCC
AGA AAA AAG AGA CUG UUC CGU AUG ACA CUG AGC GAG AAA UGU
UGU CAA GUA GGU UGU AUC AGA AAA GAU AUU GCU AGA UUA UGC

3. Doppelsträngiges DNA-Fragment, das für die Signalpeptidkette von Schweine-Präprorelaxin codiert, dadurch gekennzeichnet, daß es einen codierenden Sträng und einen komplementären Strang aufweist, entsprechend der nachfolgenden mRNA-Sequenz:

AUG CCG CGC CUG UUC UCC UAC CUC CUA GGU GUC UGG CUG CUC
CUG AGC CAA CUU CCC AGA GAA AUC CCA GGC

4. Doppelsträngiges DNA-Fragment, das für die C-Peptidkette von Schweine-Präprorelaxin codiert, dadurch gekennzeichnet, daß es einen codierenden Strang und einen komplementären Strang aufweist, entsprechend der nachfolgenden mRNA-Sequenz:

AGC CUG CAA GAG CCU CAG CUG GAA ACU GGA CCC CCG GCA GAA
ACC AUG CCA UCC UCC AUC ACC AAA GAU GCA GAA AUC UUA AAG
AUG AUG UUG GAA UUU GUU CCU AAU UUG CCA CAG GAG CUG AAG
GCA ACA UUG UGU GAG AGG CAA CCA UCA CUG AGA GAG CUA CAA
CAA UCA GCA UCA AAG GAU UCG AAU CUU AAC UUU GAA GAA UUU
AAG AAA AUU AUU CUU AAC AGA CAA AAU GAA GCA GAA GAC AAA
AGU CUU UUA GAA UUA AAA AAC UUA GGU UUA GAU AAA CAU UCC
AGA AAA AAG AGA CUG UUC

5. DNA-Fragment, das eine allelische Variente der in einem der Ansprüche 1, 2, 3 und 4 beansprüchten DNA-Fragmente codiert.

6. DNA-Fragment, das für die Signal- oder C-Peptidketten von Schweine-Präprorelaxin oder irgendeine Kombination von zwei oder mehr der Signal-, A-, B- oder C-Peptidketten von Schweine-Präprorelaxin, ausschließlich der A plus B-Kombination, codiert, wobei die Peptidketten so sind, wie in den vorangehenden Ansprüchen definiert.

7. Verfahren zum Herstellen eines DNA-Fragments nach einem der vorangehenden Ansprüche, dadurch gekennzeichnet, daß es den Einsatz von rekombinaten DNA-Techniken, um eine cDNA-Klonbank aus der gesamten mRNA zu gewinnen, welche aus Corpora lutea vom Schwein isoliert worden ist, und das Screening der Klonbank unter Verwendung einer cDNA-Sonde, die durch das synthetische Oligonukleotid [5'ATCTCCACCCA3'] initiiert wird, umfaßt.

8. DNA-Transfervektor, dadurch gekennzeichnet, daß er eine cDNA-Desoxynukleotidsequenz enthält, die einem DNA-Fragment nach einem der Ansprüche 1 bis 6 entspricht.

9. DNA-Fragment oder DNA-Transfervektor wie in einem der Ansprüche 1 bis 6 und 8 beansprucht, dadurch gekennzeichnet, daß ein oder mehrere natürliche Codons oder ihre cDNA-Aquivalente durch einen anderen Codon ersetzt sind, der für dieselbe Aminosäure codiert.

10. DNA-Transfervektor nach Anspruch 8 oder 9, dadurch gekennzeichnet, daß er ein bakterielles Plasmid ist.

11. DNA-Transfervektor nach Anspruch 8 oder 9, dadurch gekennzeichnet, daß er eine Bakteriophagen-DNA ist.

12. Durch einen Transfervektor nach Anspruch 8 oder 9 transformierte Zelle.

13. Verfahren zum Herstellen eines DNA-Transfervektors zur Verwendung für das Erhalten und die Replikation eines DNA-Fragments, das für Schweine-Präprorelaxin codiert nach einem der Ansprüche 1, 5 und 9, dadurch gekennzeichnet, daß es das Umsetzen der geeigneten Desoxynukleotidsequenz von Schweine-Präprorelaxin oder allelischen Variante mit einem DNA-Molekül, das durch Schneiden eines Transfervektors mit einem Restriktionsenzym hergestellt ist, umfaßt.

14. Verfahren zum Herstellen eines DNA-Transfervektors nach Anspruch 8 oder 9, zur Verwendung für das Erhalten und die Replikation eines DNA-Fragments, das für Schweine-Prorelaxin codiert, dadurch gekennzeichnet, daß es das Umsetzen des geeigneten DNA-Fragments mit einem DNA-Molekül, das durch Schneiden eines Transfervektors mit einem Restriktionsenzym hergestellt ist, umfaßt.

15. Verfahren zum Herstellen eines DNA-Transfervektors nach Anspruch 8 oder 9, zur Verwendung für das Erhalten und die Replikation eines DNA-Fragments, dans für das Signalpeptid oder das C-Kettenpeptid von Schweine-Präprorelaxin oder irgendeine Kombination von zwei oder mehr der Signal, A, B- oder C-Kettenpeptide, mit Ausnahme der A plus B-Kettenkombination, codiert, dadurch gekennzeichnet, daß es das Umsetzen des geeigneten DNA-Fragments mit einem DNA-Molekül, das durch Schneiden eines Transfervektors mit einem Restriktionsenzym hergestellt ist, umfaßt.

16. Schweine-Präprorelaxin, gekennzeichnet durch die Aminosäuresequenz

Met Pro Arg Leu Phe Ser Tyr Leu Leu Gly Val Trp Leu Leu Leu Ser Gln Leu Pro Arg Glu Ile Pro
Gly Gln Ser Thr Asn Asp Phe Ile Lys Ala Cys Gly Arg Glu Leu Val Arg Leu Trp Val Glu Ile Cys
Gly Ser Val Ser Trp Gly Arg Thr Ala Leu Ser Leu Glu Glu Pro Gln Leu Glu Thr Gly Pro Pro Ala
Glu Thr Met Pro Ser Ser Ile Thr Lys Asp Ala Glu Ile Leu Lys Met Met Leu Glu Phe Val Pro Asn
Leu Pro Gln Glu Leu Lys Ala Thr Leu Ser Glu Arg Gln Pro Ser Leu Arg Glu Leu Gln Gln Ser Ala
Ser Lys Asp Ser Asn Leu Asn Phe Glu Glu Phe Lys Lys Ile Ile Leu Asn Arg Gln Asn Glu

12

Ala Glu Asp Lys Ser Leu Leu Glu Leu Lys Asn Leu Gly Leu Asp Lys His Ser Arg Lys Lys Arg Leu
Phe Arg Met Thr Leu Ser Glu Lys Cys Cys Gln Val Gly Cys Ile Arg Lys Asp Ile Ala Arg Leu Cys

17. Schweine-Prorelaxin, gekennzeichnet durch die Aminosäuresequenz

Gln Ser Thr Asn Asp Phe Ile Lys Ala Cys Gly Arg Glu Leu Val Arg Leu Trp Val Glu Ile Cys
Gly Ser Val Ser Trp Gly Arg Thr Ala Leu Ser Leu Glu Glu Pro Gln Leu Glu Thr Gly Pro Pro Ala
Glu Thr Met Pro Ser Ser Ile Thr Lys Asp Ala Glu Ile Leu Lys Met Met Leu Glu Phe Val Pro Asn
Leu Pro Gln Glu Leu Lys Ala Thr Leu Ser Glu Arg Gln Pro Ser Leu Arg Glu Leu Gln Gln Ser Ala
Ser Lys Asp Ser Asn Leu Asn Phe Glu Glu Phe Lys Lys Ile Ile Leu Asn Arg Gln Asn Glu
Ala Glu Asp Lys Ser Leu Leu Glu Leu Lys Asn Leu Gly Leu Asp Lys His Ser Arg Lys Lys Arg Leu
Phe Art Met Thr Leu Ser Glu Lys Cys Cys Gln Val Gly Cys Ile Arg Lys Asp Ile Ala Arg Leu Cys

18. Verfahren zum Herstellen eines Fusionsproteins, das eine Aminosäuresequenz umfaßt, die aus der Aminosäuresequenz von Schweine-Präprorelaxin nach Anspruch 16 als deren C-terminale Sequenz und einem Abschnitt eines prokaryontischen Proteins als deren N-terminale Sequenz besteht, dadurch gekennzeichnet, daß es das Inkubieren eines mit einem Expressions-Transfervektor nach den Ansprüchen 8 oder 9, der das geeignete DNA-Fragment nach den Ansprüche 1 oder 5 umfaßt, tansformierten Mikroorganismus umfaßt.

19. Verfahren zum Synthetisieren von Schweine-Prorelaxin nach Anspruch 17, das die voneinander durch ein C-Peptid getrennten A- und B-Peptide umfaßt, dadurch gekennzeichnet, daß es das Inkubieren eines mit einem Expressions-Transfervektor nach den Ansprüchen 8 oder 9, der ein DNA-Fragment umfaßt, das für besagtes Schweine-Prorelaxin nach den Ansprüchen 2 oder 5 codiert, transformierten Mikroorganismus unter für die Expression besagter Sequenz, die für Schweine-Prorelaxin codiert, geeigneten Bedingungen, und das Reinigen des Schweine-Prorelaxins aus dem Lysat oder Kulturmedium des besagten Mikroorganismus umfaßt.

20. Fusionsprotein mit einer Aminosäuresequenz, dadurch gekennzeichnet, daß es aus der gesamten oder einem Teil der Aminosäuresequenz von Schweine-Präprorelaxin nach Anspruch 16 als deren C-terminale Sequenz und einem Abschnitt des prokaryontischen Proteins als deren N-terminale Sequenz besteht, mit dem Vorbehalt, daß die Aminosäuresequenz nicht ausschließlich die A-Ketten- oder B-Kettensequenz von Schweine-Relaxin oder die A plus B-Kettenkombination ist.

21. Synthetische Signalpeptidkette von Schweine-Prorelaxin mit der Sequenz

Met Pro Arg Leu Phe Ser Tyr Leu Leu Gly Val Trp Leu
Leu Leu Ser Gln Leu Pro Arg Glu Ile Pro Gly.

22. Synthetische C-Peptidkette von Schweine-Relaxin mit der Sequenz

Ser Leu Glu Glu Pro Gln Leu Glu Thr Gly Pro Pro Ala
Glu Thr Met Pro Ser Ser Ile Thr Lys Asp Ala Glu Ile Leu Lys Met Met Leu Glu Phe Val Pro Asn
Leu Pro Gln Glu Leu Lys Ala Thr Leu Ser Glu Arg Glu Pro Ser Leu Arg Glu Leu Gln Gln Ser Ala
Ser Lys Asp Ser Asn Leu Asn Phe Glu Glu Phe Lys Lys Ile Ile Leu Asn Arg Gln Asn Glu
Ala Glu Asp Lys Ser Leu Leu Glu Leu Lys Asn Leu Gly Leu Asp Lys His Ser Arg Lys Lys Arg Leu
Phe

## Revendications

1. Un fragment d'ADN codant pour la préprorelaxine porcine, caractérisé en ce qu'il comprend un brin codant et un brin complémentaire correspondant à la séquence d'ARNm complète suivante

AUG CCG CGC CUG UUC UCC UAC CUG CUA GGU GUC UGG CUG CUC
CUG AGC CAA CUU CCC AGA GAA AUC CCA CGC CAG AGU ACG AAC
GAU UUU AUU AAG GCA UGC GGC CGA GAA UUA GUC CGU CUG UGG
GUG GAG AUC UGU GGC UCC GUC UCC UGG GGA AGA ACU GCU CUC
AGC CUG GAA GAG CCU CAG CUG GAA ACU GGA CCC CCG GCA GAA
ACC AUG CCA UCC UCC AUC ACC AAA GAU GCA GAA AUC UUA AAG
AUG AUG UUG GAA UUU GUU CCU AAU UUG CCA CAG GAG CUG AAG
GCA ACA UUG UCU GAG AGG CAA CCA UCA CUG AGA GAG CUA CAA
CAA UCA GCA UCA AAG GAU UCG AAU CUU AAC UUU GAA GAA UUU
AAG AAA AUU AUU CUU AAC AGA CAA AAU GAA GCA CAA GAC AAA
AGU CUU UUA GAA UUA AAA AAC UUA GGU UUA GAU AAA CAU UUC
AGA AAA AAG AGA CUG UUC CGU AUG ACA CUG AGC GAG AAA UGU
UGU CAA GUA GGU UGU AUC ACA AAA GAU AUU GCU AGA UUA UGC
UGAAAAGGAGCUAAUGAUAUAUUUUAUAUAAUGUUCACAUGUAUUCUCAGUGACA
UAUUCACUUAUGCCUCUGUCACCCACUGAUUAUUAGCACUGUUUAGUGUUUAGGU
UUUUCAUUUUAUGUGUAAGAAAAUGUCCCUUGCAUUAAUGUAGUUUCUGCUAAUA
AUAUUUUUUUAAACUAAAAGGUAAAAAAAAAAAAAAAAAA.

2. Fragment d'ADN codant pour la prorelaxine porcine, caractérisé en ce qu'il comprend un brin codant et un brin complémentaire correspondant à la séquence d'ARNm suivante

CAG AGU ACG AAC
GAU UUU AUU AAG GCA UGC GGC CGA GAA UUA GUC CGU CUG UGG
GUG CAG AUC UGU GGC UCC GUC UCC UGG GGA AGA ACU GCU CUC

AGC CUG GAA GAG CCU CAG CUG GAA ACU GGA CCC CCG GCA GAA
ACC AUG CCA UCC UCC AUC ACC AAA GAU GCA GAA AUC UUA AAG
AUG AUG UUG GAA UUU GUU CCU AAU UUG CCA CAG GAG CUG AAG
GCA ACA UUG UCU GAG AGG CAA CCA UCA CUG AGA GAG CUA CAA
CAA UCA GCA UCA AAG GAU UCG AAU CUU AAC UUU GAA GAA UUU
AAG AAA AUU AUU CUU AAC AGA CAA AAU GAA GCA GAA GAC AAA
AGU CUU UUA GAA UUA AAA AAC UUA GGU UUA GAU AAA CAU UCC
AGA AAA AAG AGA CUG UUG CGU AUG ACA CUG AGC GAG AAA UGU
UGU CAA GUA GCU UGU AUC AGA AAA GAU AUU GCU AGA UUA UGC

3. Un fragment d'ADN bicaténaire codant pour la chaîne peptidique de signal de la préprorelaxine porcine, caractérisé en ce qu'il comprend un brin codant et un brin complémantaire correspondant à la séquence d'ARNm suivante

AUG CCG CGC CUG UUC UCC UAC CUC CUA GGU GUC UGG CUG CUC
CUG AGC CAA CUU CCC AGA GAA AUC CCA GGC.

4. Fragment d'ADN bicaténaire codant pour la chaîne peptidique C de la préprorelaxine porcine, caractérisé en ce qu'il comprend un brin codant et un brin complémentaire correspondant à la séquence d'ARNm suivante

AGC CUG GAA GAG CCU CAG CUG GAA ACU GGA CCC CCG GCA GAA
ACC AUG CCA UCC UCC AUC ACC AAA GAU GCA GAA AUC UUA AAG
AUG AUG UUG GAA UUU GUU CCU AAU UUG CCA CAG GAG CUG AAG
GCA ACA UUG UCU GAG AGG CAA CCA UCA CUG AGA GAG CUA CAA
CAA UCA GCA UCA AAG GAU UCG AAU CUU AAC UUU GAA GAA UUU
AAG AAA AUU AUU CUU AAC AGA CAA AAU GAA GCA GAA GAG AAA
AGU CUU UUA GAA UUA AAA AAC UUA GGU UUA CAU AAA CAU UCC
AGA AAA AAG AGA CUG UUC.

5. Fragment d'ADN condant pour un variant allélique des fragments d'ADN tels que définis dans l'une quelconque des revendications 1, 2, 3 et 4.

6. Fragment d'ADN codant pour la chaîne peptidique de signal ou la chaîne peptidique C de la préprorelaxine porcine ou n'importe quelle combinaison de deux ou plusieurs des chaînes peptidiques de signal, A, B et C de la préprorelaxine porcine, à l'exclusion de la combinaison A plus B, les chaînes peptidiques étant définies dans les revendications précédentes.

7. Un procédé pour la production d'un fragment d'ADN comme défini dans l'une quelconque des revendications précédentes, caractérisé en ce qu'il comprend l'utilisation des techniques d'ADN recombinant pour obtenir un ensemble de clones d'ADNc à partir d'ARNm total, isolé à partir de corps jaune de porcs, et le screening de l'ensemble des clones en utilisant une sonde d'ADNc initié par l'oligonucléotide synthétique [5'ATCTCCACCCA3'].

8. Un vecteur de transfert d'ADN, caractérisé en ce qu'il contient des séquences de désoxynucléotides ADNc correspondant à un fragment d'ADN tel que défini dans l'une quelconque des revendications 1 à 6.

9. Un fragment d'ADN ou un vecteur de transfert d'ADN suivant l'une quelconque des revendications 1 à 6 et 8, caractérisé en ce que un ou plusieurs codons naturels ou leurs équivalents ADNc sont remplacés par un autre codon qui code pour le même amino-acide.

10. Un vecteur de transfert d'ADN selon la revendication 8 ou 9, caractérisé en ce qu'il est un plasmide bactérien.

11. Un vecteur de transfert d'ADN selon la revendication 8 ou 9 qui est un ADN de bactériophage.

12. Une cellule transformée par un vecteur de transfert selon la revendication 8 ou 9.

13. Un procédé d'obtention d'un vecteur de transfert d'ADN destiné à maintenir et à répliquer un fragment d'ADN codant pour la préprorelaxine porcine tel que défini par l'une quelconque des revendications 1, 5 et 9, caractérisé en ce qu'il comprend la réaction de la séquence de désoxynucléotides appropriée de préprorelaxine porcine ou d'un variant allélique avec une molécule d'ADN préparée par coupure d'un vecteur de transfert avec une enzyme de restriction.

14. Un procédé d'obtention d'un vecteur de transfert d'ADN selon la revendication 8 ou 9 destiné à maintenir et à répliquer un fragment d'ADN codant pour la prorelaxine porcine, caractérisé en ce qu'il comprend la réaction du fragment d'ADN approprié de la prorelaxine porcine avec une molécule d'ADN préparée par coupure d'un vecteur de transfert avec une enzyme de restriction.

15. Un procédé d'obtention d'un vecteur de transfert d'ADN tel que défini dans la revendication 8 ou 9 destiné à maintenir et à répliquer un fragment d'ADN codant pour le peptide de signal ou le peptide de la chaîne C de la préprorelaxine porcine ou l'une quelconque des combinaisons de deux ou plusieurs des chaînes peptidiques de signal A, B ou C, à l'exclusion de la combinaison des chaînes A plus B, caractérisé en ce qu'il comprend la réaction du fragment d'ADN approprié avec une molécule d'ADN préparée par coupure d'un vecteur de transfert avec une enzyme de restriction.

16. Préprorelaxine porcine ayant la séquence d'amino acides.

Met Pro Arg Leu Phe Ser Tyr Leu Leu Gly Val Trp Leu Leu Leu Ser Gln Leu Pro Arg Glu Ile Pro
Gly Gln Ser Thr Asn Asp Phe Ile Lys Ala Cys Gly Arg Glu Leu Val Arg Leu Trp Val Glu Ile Cys
Gly Ser Val Ser Trp Gly Arg Thr Ala Leu Ser Leu Glu Glu Pro Gln Leu Glu Thr Gly Pro Pro Ala
Glu Thr Met Pro Ser Ser Ile Thr Lys Asp Ala Glu Ile Leu Lys Met Met Leu Glu Phe Val Pro Asn

Leu Pro Gln Glu Leu Lys Ala Thr Leu Ser Glu Arg Gln Pro Ser Leu Arg Glu Leu Gln Gln Ser Ala
Ser Lys Asp Ser Asn Leu Asn Phe Glu Glu Phe Lys Lys Ile Ile Leu Asn Arg Gln Asn Glu
Ala Glu Asp Lys Ser Leu Leu Glu Leu Lys Asn Leu Gly Leu Asp Lys His Ser Arg Lys Lys Arg Leu
Phe Arg Met Thr Leu Ser Glu Lys Cys Cys Gln Val Gly Cys Ile Arg Lys Asp Ile Ala Arg Leu Cys

17. Prorelaxine porcine ayant la séquence d'amino-acides.
Gln Ser Thr Asn Asp Phe Ile Lys Ala Cys Gly Arg Glu Leu Val Arg Leu Trp Val Glu Ile Cys
Gly Ser Val Ser Trp Gly Arg Thr Ala Leu Ser Leu Glu Glu Pro Gln Leu Glu Thr Gly Pro Pro Ala
Glu Thr Met Pro Ser Ser Ile Thr Lys Asp Ala Glu Ile Leu Lys Met Met Leu Glu Phe Val Pro Asn
Leu Pro Gln Glu Leu Lys Ala Thr Leu Ser Glu Arg Gln Pro Ser Leu Arg Glu Leu Gln Gln Ser Ala
Ser Lys Asp Ser Asn Leu Asn Phe Glu Glu Phe Lys Lys Ile Ile Leu Asn Arg Gln Asn Glu
Ala Glu Asp Lys Ser Leu Leu Glu Leu Lys Asn Leu Gly Leu Asp Lys His Ser Arg Lys Lys Arg Leu
Phe Arg Met Thr Leu Ser Glu Lys Cys Cys Gln Val Gly Cys Ile Arg Lys Asp Ile Ala Arg Leu Cys

18. Un procédé d'obtention d'une protéine de fusion comprenant une séquence d'amino-acides consistant en la séquence d'amino-acides de la préprorelaxine porcine telle que définie dans la revendication 16 comme séquence C-terminale et en une portion d'une protéine procaryotique comme séquence N-terminale, caractérisé en ce qu'il comprend l'incubation d'un microorganisme transformé par un vacteur de transfert d'expression tel que défini dans la revendication 8 ou 9, comprenant le fragment d'ADN approprié tel que défini dans la revendication 1 ou 5.

19. Un procédé de synthése de prorelaxine porcine selon la revendication 17, comprenant les peptides A et B séparés l'un du l'autre par un peptide C, caractérisé en ce qu'il comprend l'incubation d'un microorganisme transformé par un vecteur de transfert d'expression tel que défini dans la revendication 8 ou 9, comprenant un fragment d'ADN codant pour ladite prorelaxine porcine tel que défini dans la revendication 2 ou 5 dans des conditions appropriées pour l'expression de ladite séquence codant pour la prorelaxine porcine, et la purification de la prorelaxine porcine à partir du lysat ou du milieu de culture dudit microorganisme.

20. Une protéine de fusion comprenant une séquence d'amino-acides caractérisé en ce qu'elle consiste en totalité ou en partie en la séquence d'amino-acides de la préprorelaxine porcine telle que définie dans la revendication 16 commé séquence C-terminale et en une portion d'une protéine procaryotique comme séquence N-terminale sous réserve que la séquence d'amino-acides n'est pas uniquement la séquence de la chaîne à ou de la chaîne B de la relaxine porcine ou la combinaison des chaînes A plus B.

21. La chaîne peptidique de signal obtenue par la synthèse de la prorelaxine porcine ayant la séquence.
Met Pro Arg Leu Phe Ser Tyr Leu Leu Gly Val Trp Leu
Leu Leu Ser Gln Leu Pro Arg Glu Ile Pro Gly.

22. La chaîne peptidique C obtenue par synthèse de relaxine porcine ayant la séquence.
Ser Leu Glu Glu Pro Gln Leu Glu Thr Gly Pro Pro Ala
Glu Thr Met Pro Ser Ser Ile Thr Lys Asp Ala Glu Ile Leu Lys Met Met Leu Glu Phe Val Pro Asn
Leu Pro Gln Glu Leu Lys Ala Thr Leu Ser Glu Arg Gln Pro Ser Leu Arg Glu Leu Gln Gln Ser Ala
Ser Lys Asp Ser Asn Leu Asn Phe Glu Glu Phe Lys Lys Ile Ile Leu Asn Arg Gln Asn Glu
Ala Glu Asp Lys Ser Leu Leu Glu Leu Lys Asn Leu Gly Leu Asp Lys His Ser Arg Lys Lys Arg Leu
Phe

EP 0 086 649 B1

a.    Val[15]   Arg   Leu   Trp   Val   Glu   Ile   Cys   Gly

b.   5'– GUX   $\frac{C}{A}$GX   $\frac{U}{C}$UX   UGG   GUX   GA$\frac{A}{G}$   AUC$\frac{U}{A}$   UG$\frac{U}{C}$   GGX –3'

                             |||     |||    |||    ||

c.             3'   ACC   CAC   CTC   TA – 5'

*FIG. 1.*

FIG.2.

5′

3′
PstI

TaqI
HinfI

BglII

HpaII

Signal Bchain

C region

A chain

pRLX2

pRLX14

pRLX13

a

b

i

j

group A

c

d

e

f

g

h

k

m

group B

l

n

q

o

p

Fig.3.

| | |
|---|---|
| FIG.4A | FIG.4B |
| FIG.4C | FIG.4D |

FIG.4.

| |
|---|
| FIG.5A |
| FIG.5B |

FIG.5.

4

Signal peptide ⟶

|  |  |  | -20 |  |  |  |  |  |  |  |  |  |
|---|---|---|---|---|---|---|---|---|---|---|---|---|
| Met | Pro | Arg |  | Leu | Phe | Ser | Tyr | Leu | Leu | Gly | Val | Trp Leu |
| AUG | CCG | CGC |  | CUG | UUC | UCC | UAC | CUC | CUA | GGU | GUC | UGG CUG |
| *** | * | ** |  | ** | ** | * | * | *** | ** | ** | ** | *** *** |
| AUG | UCC | AGC | AGA | CUC | UUG | CUC | CAG | CUC | CUG | GGG | UUC | UGG CUG |
| Met | Ser | Ser | Arg | Leu | Leu | Leu | Gln | Leu | Leu | Gly | Phe | Trp Leu |
|  |  | -20 |  |  |  |  |  |  |  |  |  | -10 |

|  |  |  |  |  |  |  | 10 |  |  |  |  |  |
|---|---|---|---|---|---|---|---|---|---|---|---|---|
| Ser | Thr | Asn | Asp | Phe | Ile | Lys | Ala | Cys | Gly | Arg | Glu | Leu Val |
| AGU | ACG | AAC | GAU | UUU | AUU | AAG | GCA | UGC | GGC | CGA | GAA | UUA GUC |
| * | * * | ** | * | * | *** | ** | * | *** | *** | ** | * * | * * |
| UGG | AUG | GAC | CAA | GUC | AUU | CAG | GUG | UGC | GGC | CGU | GGA | UAU GCC |
| Trp | Met | Asp | Gln | Val | Ile | Gln | Val | Cys | Gly | Arg | Gly | Tyr Ala |

C chain ⟶

|  | 30 |  |  |  |  |  |  |  |  |  | 40 |  |
|---|---|---|---|---|---|---|---|---|---|---|---|---|
| Arg | Thr | Ala | Leu | Ser | Leu | Glu | Glu | Pro |  | Gln | Leu | Glu Thr |
| AGA | ACU | GCU | CUC | AGC | CUG | GAA | GAG | CCU. |  | CAG | CUG | GAA ACU |
| *** |  | *** | * | *** | * * | ** | *** | ** |  | * * | ** | * |
| AGA | CUG | GCU | UUG | AGC | CAG | GAG | GAG | CCA | GCU | CCG | CUA | GCC AGG |
| Arg | Leu | Ala | Leu | Ser | Gln | Glu | Glu | Pro | Ala | Pro | Leu | Ala Arg |

|  |  |  | 60 |  |  |  |  |  |  |  |  |  |
|---|---|---|---|---|---|---|---|---|---|---|---|---|
| Asp | Ala | Glu | Ile | Leu | Lys | Met | Met | Leu | Glu | Phe | Val | Pro Asn |
| GAU | GCA | GAA | AUC | UUA | AAG | AUG | AUG | UUG | GAA | UUU | GUU | CCU AAU |
| *** | ** | ** |  | ** | * | *** | * * | *** | ** | * | ** | ** *** |
| GAU | GCG | GAG | CCU | UUC | GAU | AUG | ACG | UUG | AAA | UGC | CUU | CCA AAU |
| Asp | Ala | Glu | Pro | Phe | Asp | Met | Thr | Leu | Lys | Cys | Leu | Pro Asn |

*FIG. 4A.*

B chain

-10          -1   1

| Leu | Leu | Ser | Gln | Leu | Pro | Arg | Glu | | Ile | Pro | Gly | Gln |
| CUC | CUG | AGC | CAA | CUU | CCC | AGA | GAA | | AUC | CCA | GGC | CAG |
| ** | *** | *** | ** | * * | * | ** | * | | ** | * | * | ** |
| UUC | CUG | AGC | CAG | CCU | UGC | AGG | GCG | CGA | GUC | UCG | GAG | GAG |
| Phe | Leu | Ser | Gln | Pro | Cys | Arg | Ala | Arg | Val | Ser | Glu | Glu |

-1   1

20

| Arg | Leu | Trp | Val | Glu | Ile | Cys | Gly | Ser | Val | Ser | Trp | Gly |
| CGU | CUG | UGG | GUG | GAG | AUC | UGU | GGC | UCC | GUC | UCC | UGG | GGA |
| ** | | *** | * | ** | ** | ** | ** | ** | | *** | * | *** |
| CGC | GCA | UGG | AUC | GAA | GUC | UGC | GGG | CCC | | UCC | GUG | GGA |
| Arg | Ala | Trp | Ile | Glu | Val | Cys | Gly | Ala | | Ser | Val | Gly |

50

| Gly | Pro | Pro | Ala | Glu | Thr | Met | Pro | Ser | Ser | Ile | Thr | Lys |
| GGA | CCC | CCG | GCA | GAA | ACC | AUG | CCA | UCC | UCC | AUC | ACC | AAA |
| * | ** | * | *** | *** | | ** | *** | *** | * * | *** | * * | *** |
| CAA | GCC | ACU | GCA | GAA | GUU | GUG | CCA | UCC | UUC | AUC | AAC | AAA |
| Gln | Ala | Thr | Ala | Glu | Val | Val | Pro | Ser | Phe | Ile | Asn | Lys |

*    50

70                      80

| Leu | Pro | Gln | Glu | Leu | Lys | Ala | Thr | Leu | Ser | Glu | Arg | Gln |
| UUG | CCA | CAG | GAG | CUG | AAG | GCA | ACA | UUG | UCU | GAG | AGG | CAA |
| *** | * | ** | *** | * * | *** | *** | ** | ** | *** | *** | ** | * * |
| UUG | UCU | GAG | GAG | CGG | AAG | GCA | GCA | CUG | UCU | GAG | GGG | CGA |
| Leu | Ser | Glu | Glu | Arg | Lys | Ala | Ala | Leu | Ser | Glu | Gly | Arg |

FIG. 4B.

```
                                                     90
Pro  Ser  Leu  Arg │Glu  Leu  Gln  Gln│ Ser │Ala│ Ser  Lys
CCA  UCA  CUG  AGA │GAG  CUA  CAA  CAA│ UCU │GCA│ UCA  AAG
 **   *    *    *  │***  ***  ***  ***│  *  │***│  *    **
GCA  CCG  UUC  CCA │GAG  CUA  CAA  CAA│ CAC │GCA│ CCC  GCG  UUG  AGC
Ala  Pro  Phe  Pro │Glu  Leu  Gln  Gln│ His │Ala│ Pro  Ala  Leu  Ser


                    110                                        120
Leu │Asn│ Arg  Gln  Asn │Glu  Ala  Glu  Asp│ Lys  Ser  Leu  Leu │Glu
CUU │AAC│ AGA  CAA  AAU │GAA  GCA  GAA  GAC│ AAA  AGU  CUU  UUA │GAA
 *  │ **│  *         *  │***  ***  ***  ** │      **   *    *  │ **
CAC │AAU│ CAG  CUG  GGU │GAA  GCA  GAA  GAU│ GGC  GGU  CCU  CCA │GAG
His │Asn│ Gln  Leu  Gly │Glu  Ala  Glu  Asp│ Gly  Gly  Pro  Pro │Glu


               A chain
               ┌────────►
               │                    140
Arg │Leu  Phe │Arg  Met  Thr │Leu  Ser  Glu│ Lys │Cys  Cys│ Gln  Val
AGA │CUG  UUC │CGU  AUG  ACA │CUG  AGC  GAG│ AAA │UGU  UGU│ CAA  GUA
 ** │ *    *  │      *        │ **   **  ***│  *  │***  ** │ **    *
AGG │CAG  UCU │GGC  GCA  CUG │CUC  AGU  GAG│ CAG │UGU  UGC│ CAC  AUC
Arg │Gln  Ser·│Gly  Ala  Leu │Leu  Ser  Glu│ Gln │Cys  Cys│ His  Ile
                                                   150


┌──────────────┐
│AAAGGAGCUAAUG│AUAUAUUUUAUAUAAUGUUCACAUGUAUUCUCAGUGACAUAUUC
│ *  ********* │
│UACGGAGCUAAUG│
└──────────────┘


GCACUGUUUAGUGUUUAGGUUUUUCAUUUUAUGUGUAAGAAAAUGUCCCUUGCAUUAA


ACUAAAAGGUAAAAAAAAAAAAAAAAAAAAA
```

*FIG. 4C.*

100

| Asp | Ser | Asn | Leu | Asn | Phe | Glu | Glu | Phe | Lys | Lys | Ile | Ile |
|-----|-----|-----|-----|-----|-----|-----|-----|-----|-----|-----|-----|-----|
| GAU | UCG | AAU | CUU | AAC | UUU | GAA | GAA | UUU | AAG | AAA | AUU | AUU |
| *** | ** | * | * | * | ** | *** | * * | *** | *** | *** | * * | * |
| GAU | UCC | GUU | GUU | AGG | UUG | GAA | GGA | UUU | AAG | AAA | ACU | UUC |
| Asp | Ser | Val | Val | Ser | Leu | Glu | Gly | Phe | Lys | Lys | Thr | Phe |

100

130

| Leu | Lys | Asn | Leu | Gly | Leu | Asp | Lys | His | Ser | Arg | Lys | Lys |
|-----|-----|-----|-----|-----|-----|-----|-----|-----|-----|-----|-----|-----|
| UUA | AAA | AAC | UUA | GGU | UUA | GAU | AAA | CAU | UCC | AGA | AAA | AAG |
| * | *** | ** | *** | ** | * * | *** | | ** | ** | * | *** | *** |
| CUC | AAA | UAC | UUA | GGC | UCA | GAU | GCU | CAG | UCA | CGG | AAA | AAG |
| Leu | Lys | Tyr | Leu | Gly | Ser | Asp | Ala | Gln | Ser | Arg | Lys | Lys |

150

| Gly | Cys | Ile | Arg | Lys | Asp | Ile | Ala | Arg | Leu | Cys | *** |
|-----|-----|-----|-----|-----|-----|-----|-----|-----|-----|-----|-----|
| GGU | UGU | AUC | AGA | AAA | GAU | AUU | GCU | AGA | UUA | UGC | UGA |
| *** | *** | * * | *** | * * | | *** | *** | * * | | *** | *** |
| GGU | UGU | ACC | AGA | AGA | UCC | AUU | GCU | AAA | CUC | UGC | UGA |
| Gly | Cys | Thr | Arg | Arg | Ser | Ile | Ala | Lys | Leu | Cys | *** |

ACUUAUGCCUCUGUCACCCACUGAUUAUUA

A UGUAGUUUCUGCUAAUAAUAUUUUUUU AA
****** *** ************ *
UGUAGUU CUGUUAAUAAUAUUUUUAU

FIG. 4D.

8

```
Met Pro Ars Leu Phe Ser Tyr Leu Leu Gly Val Trp Leu Leu Leu
AUG CCG CGC CUG UUC UCC UAC CUC CUA GGU GUC UGG CUG CUC CUG
175


Ser Gln Leu Pro Ars Glu Ile Pro Gly Gln Ser Thr Asn Asp Phe
AGC CAA CUU CCC AGA GAA AUC CCA GGC CAG AGU ACG AAC GAU UUU
220


Ile Lys Ala Cys Gly Ars Glu Leu Val Ars Leu Trp Val Glu Ile
AUU AAG GCA UGC GGC CGA GAA UUA GUC CGU CUG UGG GUG GAG AUC
265


Cys Gly Ser Val Ser Trp Gly Ars Thr Ala Leu Ser Leu Glu Glu
UGU GGC UCC GUC UCC UGG GGA AGA ACU GCU CUC AGC CUG GAA GAG
310


Pro Gln Leu Glu Thr Gly Pro Pro Ala Glu Thr Met Pro Ser Ser
CCU CAG CUG GAA ACU GGA CCC CCG GCA GAA ACC AUG CCA UCC UCC
355


Ile Thr Lys Asp Ala Glu Ile Leu Lys Met Met Leu Glu Phe Val
AUC ACC AAA GAU GCA GAA AUC UUA AAG AUG AUG UUG GAA UUU GUU
400


Pro Asn Leu Pro Gln Glu Leu Lys Ala Thr Leu Ser Glu Ars Gln
CCU AAU UUG CCA CAG GAG CUG AAG GCA ACA UUG UCU GAG AGG CAA
445


Pro Ser Leu Ars Glu Leu Gln Gln Ser Ala Ser Lys Asp Ser Asn
CCA UCA CUG AGA GAG CUA CAA CAA UCU GCA UCA AAG GAU UCG AAU
490
```

# *FIG. 5A.*

---

Leu Asn Phe Glu Glu Phe Lys Lys Ile Ile Leu Asn Ars Gln Asn
CUU AAC UUU GAA GAA UUU AAG AAA AUU AUU CUU AAC AGA CAA AAU
535

Glu Ala Glu Asp Lys Ser Leu Leu Glu Leu Lys Asn Leu Gly Leu
GAA GCA GAA GAC AAA AGU CUU UUA GAA UUA AAA AAC UUA GGU UUA
580

Asp Lys His Ser Ars Lys Lys Ars Leu Phe Ars Met Thr Leu Ser
GAU AAA CAU UCC AGA AAA AAG AGA CUG UUC CGU AUG ACA CUG AGC
625

Glu Lys Cys Cys Gln Val Gly Cys Ile Ars Lys Asp Ile Ala Ars
GAG AAA UGU UGU CAA GUA GGU UGU AUC AGA AAA GAU AUU GCU AGA
670

Leu Cys ***
UUA UGC UGAAAAGGAGCUAAUGAUAUAUUUUAUAUAAUGUUCACAUGUAUUCUC
715

AGUGACAUAUUCACUUAUGCCUCUGUCACCCACUGAUUAUUAGCACUGUUUAGUGUUUA
771

GGUUUUUCAUUUUAUGUGUAAGAAAAUGUCCCUUGCAUUAAUGUAGUUUCUGCUAAUAA
830

UAUUUUUUUAAACUAAAAGGUAAAAAAAAAAAAAAAAAAAAA
889

# FIG. 5B.

|   |   | 5'  | 3' |
|---|---|-----|----|
| 1. | 544-559 | GAAGAATTTAAGAAAA | |
|   |   | CTTCTTAAATTCTTTT | |
| 2. | 458-471 | AGGAGCTGAAGGCA | |
|   |   | TCCTCGACTTCCGT | |
| 3. | 405-416 | CAAAGATGCAGA | |
|   |   | GTTTCTACGTCT | |
| 4. | 476-487 | TGTCTGAGAGGC | |
|   |   | ACAGACTCTCCG | |
| 5.. | 579-590 | TGAAGCAGAAGA | |
|   |   | ACTTCGTCTTCT | |
| 6. | 501-513 | AGAGCTACAACAA | |
|   |   | TCTCGATGTTGTT | |
| 7. | 610-620 | AAAAACTTAGG | |
|   |   | TTTTTGAATCC | |
| 8. | 688-700 | GGTTGTATCAGAA | |
|   |   | CCAACATAGTCTT | |

## FIG.6.